# FASCICULE DE BREVET EUROPEEN

(11) **EP 1 940 304 B1**
(45) Date de publication et mention de la délivrance du brevet: **22.07.2009**
(21) Numéro de dépôt: 01993431.4
(22) Date de dépôt: 05.11.2001
(51) Int. Cl.: A61B 17/70

(54) **MATERIEL D'ARTHRODESE VERTEBRALE**
WIRBELARTHRODESEVORRICHTUNG
VERTEBRAL ARTHRODESIS EQUIPMENT

(30) Priorité: 07.11.2000 FR 0014270
(43) Date de publication de la demande: 09.07.2008
(73) Titulaire: Médicréa Technologies, 17000 La Rochelle (FR)
(72) Inventeur: TAYLOR, Jean, F-06400 Cannes (FR); VILLARET, Bernard, F-17220 Croix Chapeau (FR); PARISINI, Patrizio, I-40123 Bologne (IT); CLEMENT, Jean-Luc, F-06480 La Colle Sur Loup (FR)
(74) Mandataire: Jeannet, Olivier
(86) Numéro de dépôt international: PCT/FR2001/003412
(87) Numéro de publication internationale: WO 2002/038061

(56) Documents cités:
- EP-A- 0 383 992
- WO-A-00/59387

## Description

La présente invention concerne un matériel d'arthrodèse vertébrale.

Un tel matériel comprend généralement deux tiges d'étayage, destinées à être disposées parallèlement l'une à l'autre de part et d'autre des vertèbres, et des organes d'ancrage de ces tiges aux vertèbres, tels que des crochets ou des vis pédiculaires. Ce matériel peut éventuellement comprendre en outre des traverses qui relient transversalement ces tiges de proche en proche pour les maintenir l'une par rapport à l'autre.

Les traverses existantes sont dans certains cas constituées par de simples barrettes reliées aux tiges d'étayage par des organes d'assemblage appropriés. Ces barrettes ont pour inconvénient de ne pas permettre de s'adapter à toutes les situations pouvant se présenter, en particulier de ne pas pouvoir s'adapter aux différentes positions possibles des tiges. En particulier, les tiges peuvent avoir des écartements qui varient d'un emplacement à un autre, ou des orientations dans des plans différents.

II a été conçu de réaliser des traverses en deux parties, pouvant pivoter dans un plan l'une par rapport à l'autre. Une telle traverse est connue du document EP-A- 0383992. Une traverse en deux parties pouvant pivoter autour de deux axes est connue du document WO-A- 0059387. Le préambule de la revendication 1 est basé sur cet état de la technique. Ces traverses ne remédient toutefois qu'imparfaitement aux inconvénients précités.

Les organes d'assemblage que comprennent les matériels existants pour assembler des traverses aux tiges d'étayage ne donnent pas, en outre, parfaitement satisfaction. En effet, ces organes ont soit des structures relativement complexes et difficiles à fabriquer, soit sont relativement encombrants et surélèvent excessivement une traverse par rapport aux tiges d'étayage jusqu'à risquer de rendre cette traverse sensible sous la peau, soit laissent subsister des doutes quant à la parfaite résistance dans le temps des assemblages obtenus.

La présente invention a pour objectif principal de remédier à ces inconvénients.

Par ailleurs, la fixation des matériels existants au sacrum est relativement problématique, à défaut de disposer d'organes d'ancrage parfaitement appropriés pour fixer les tiges d'étayage au sacrum.

L'invention a également pour objectif d'apporter une solution satisfaisante à ce problème.

Le matériel qu'elle concerne comprend, de manière connue en soi, deux tiges d'étayage destinées à être disposées parallèlement l'une à l'autre de part et d'autre des vertèbres à traiter, des organes d'ancrage de ces tiges aux vertèbres, tels que des crochets ou des vis pédiculaires, au moins une traverse en deux parties et des organes d'assemblage de cette traverse aux tiges d'étayage.

Selon l'invention,
- l'une des parties de traverse comprend une tête sphérique au niveau de son extrémité destinée à être reliée à l'autre partie de traverse et un filetage adjacent à cette tête ;
- l'autre partie de traverse comprend une extrémité renflée de forme sphérique, délimitant intérieurement une cavité sphérique propre à recevoir ladite tête avec possibilité de mouvements d'articulation de cette tête dans cette cavité, la paroi délimitant cette cavité étant sertie autour de ladite tête de manière à assurer la rétention de cette tête dans la cavité, et
- chaque traverse comprend un écrou propre à coopérer avec le filetage précité, présentant en outre une portée sphérique de forme correspondant à celle de ladite paroi délimitant la cavité sphérique.

La tête et la cavité sphérique forment ainsi une rotule permettant un positionnement multidirectionnel d'une partie de traverse par rapport à l'autre. La traverse selon l'invention peut ainsi s'adapter à toutes les situations susceptibles de se présenter, notamment à des orientations de tiges d'étayage dans des plans différents. Lorsque la position relative adéquate des parties de traverse est déterminée, l'écrou est vissé sur le filetage jusqu'à prendre appui contre la paroi de l'extrémité renflée et exercer une pression sur celle-ci. Cette pression crée une tension sur la tête par rapport à l'extrémité renflée et des frottements entre celles-ci, qui permettent d'immobiliser une partie de traverse par rapport à l'autre.

Avantageusement, le matériel comprend une vis pour relier une partie de traverse à l'organe permettant l'assemblage de cette partie de traverse à la tige d'étayage correspondante, et cette partie de traverse comprend une ouverture oblongue permettant une pluralité de positions de cette vis, et donc de l'organe d'assemblage, par rapport à la partie de traverse.

La traverse peut ainsi être adaptée à des écartements différents des tiges d'étayage.

De préférence, chacune des deux parties de traverse comprend une ouverture oblongue telle que précitée, ce qui augmente l'adaptabilité de la traverse à différents écartements des tiges d'étayage.

De préférence, chaque partie de traverse comprenant ladite ouverture oblongue présente une pluralité d'évidements délimitant, individuellement ou par paires, des logements circulaires propres à recevoir la tête de ladite vis.

Ces logements définissent ainsi plusieurs positions déterminées de la vis par rapport à la partie de traverse correspondante, ce qui permet d'assurer l'immobilisation de cette vis en translation dans ladite ouverture oblongue, après serrage.

Selon une forme de réalisation préférée de l'invention, chaque organe d'assemblage comprend une pièce monobloc présentant :
- un évidement aménagé dans cette pièce, à partir d'un des bords de celle-ci, qui traverse la pièce et qui délimite deux parois de serrage latérales, propres à recevoir étroitement une tige d'étayage entre elles ;
- une fente en forme de "L", aménagée au travers de cette pièce selon une même direction que ledit évidement ; une première des deux parties de fente débouche dans l'évidement, latéralement par rapport à celui-ci, et est orientée de manière sensiblement parallèle auxdites parois de serrage ; la deuxième partie de fente est orientée sensiblement perpendiculairement auxdites parois de serrage et s'étend sur une longueur relativement importante, de telle sorte que chaque paroi de serrage est reliée au reste de la pièce par une zone de largeur relativement réduite, présentant une souplesse élastique, et
- deux alésages coaxiaux, dont un premier, non taraudé, est aménagé au travers d'une première zone de la pièce, délimitée par la face de cette pièce opposée à l'évidement d'une part et par ladite deuxième partie de fente d'autre part, et dont un deuxième, taraudé, est aménagé au travers d'une deuxième zone de la pièce, délimitée par ladite deuxième partie de fente d'une part et par l'évidement d'autre part.

Une vis peut ainsi être engagée au travers d'une ouverture aménagée dans une partie de traverse, puis au travers du premier alésage et être vissée au travers du deuxième alésage. Le serrage de cette vis provoque le rapprochement de ladite deuxième zone et de ladite première zone, par déformation de la pièce. Cette déformation permet le serrage desdites parois de serrage autour d'une tige d'étayage engagée dans ledit logement.

L'organe d'assemblage ainsi conformé est à la fois simple à fabriquer, très résistant et assure un parfait serrage de la tige d'étayage.

Selon un autre aspect de l'invention, le matériel comprend deux plaquettes d'ancrage de tiges d'étayage au niveau du sacrum, comportant chacune des moyens d'assemblage à une tige d'étayage correspondante ;
- chaque plaquette présente deux trous latéraux pouvant recevoir des vis d'ancrage, l'un de ces trous ayant un axe sensiblement perpendiculaire à la face par laquelle cette plaquette est destinée à venir en appui contre le sacrum et l'autre trou ayant un axe qui forme un angle d'environ 60 degrés par rapport à cette même face ;
- les moyens d'assemblage que comporte chaque plaquette comprennent :
   (i) un bossage solidaire de la plaquette, délimitant intérieurement, avec la plaquette, une cavité de forme sphérique et délimitant extérieurement une portion de paroi sphérique ; les centres générant la sphère creuse qui forme la cavité et ladite portion de paroi sphérique sont décalés l'un par rapport à l'autre selon une direction perpendiculaire à la plaquette ;
   (ii) une tige filetée comprenant une tête sphérique destinée à être engagée et à être retenue dans ladite cavité de la plaquette, cette tête étant propre à coopérer avec la cavité de manière à permettre l'articulation de la tige filetée par rapport à la plaquette ;
   (iii) un étrier présentant une partie arrondie propre à recevoir une tige d'étayage, deux oeillets superposés percés de trous pour permettre l'engagement de cet étrier sur ladite tige filetée, et une face en portion de sphère creuse, cette face étant destinée à venir porter contre ladite portion de paroi sphérique lorsque l'étrier est engagé sur ladite tige filetée, et
   (iv) un écrou pouvant être vissé sur la tige filetée de manière à permettre le serrage de l'étrier entre lui et ladite portion de paroi sphérique.

Grâce à la cavité et à la tête sphérique, la tige filetée peut, au moment de la mise en place du matériel, être orientée en direction de l'étrier, lui-même préalablement engagé autour de la tige d'étayage. Cette orientation permet de faciliter l'engagement des oeillets de l'étrier sur cette tige filetée.

L'écrou peut alors être engagé sur la tige filetée et être vissé, ce qui a pour effet d'amener ladite face concave de l'étrier au contact de ladite portion de paroi sphérique du bossage puis, une fois ce contact intervenu, et compte tenu du décalage des deux centres précités, d'amener progressivement, au fur et à mesure du serrage de l'écrou, ladite tige filetée dans une direction sensiblement perpendiculaire à la plaquette.

Ces moyens d'assemblage facilitent ainsi la mise en place du matériel et permettent une venue progressive de la tige d'étayage en position par rapport aux plaquettes d'ancrage.

Pour sa bonne compréhension, l'invention est à nouveau décrite ci-dessous en référence au dessin schématique annexé représentant, à titre d'exemple non limitatif, une forme de réalisation préférée du matériel qu'elle concerne.
La figure 1 est une vue en perspective éclatée d'une traverse, de deux pièces d'assemblage et de deux vis qu'il comprend ;
la figure 2 est une vue en perspective d'une plaquette d'ancrage du matériel au sacrum ;
la figure 3 est une vue de cette plaquette en coupe selon la ligne IV-IV de la figure 2, et
la figure 4 est une vue des plaquettes et du reste du matériel, après mise en place sur le sacrum.

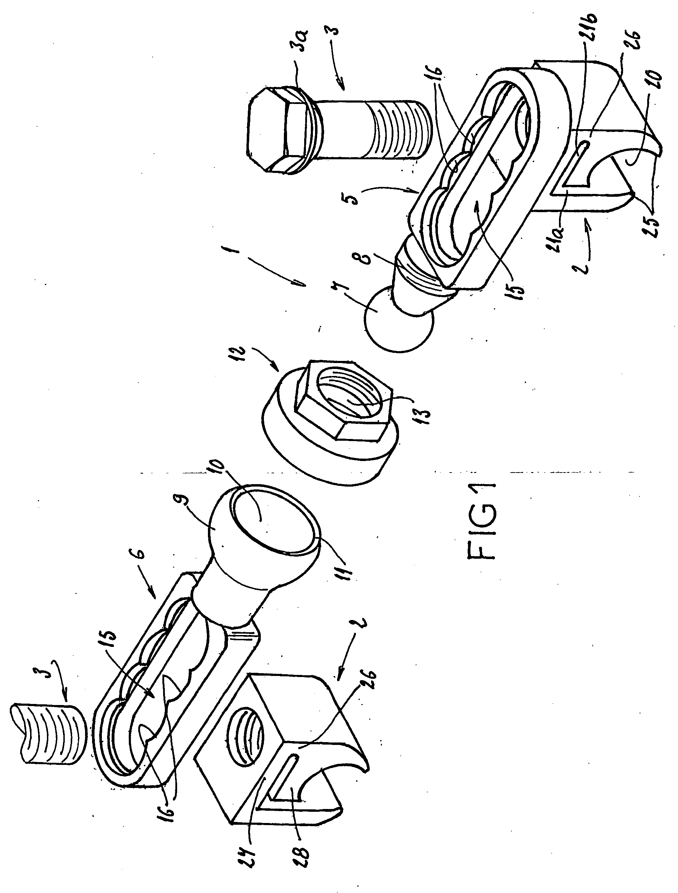

La figure 1 représente une traverse 1, des pièces 2 et des vis 3 faisant partie d'un matériel d'arthrodèse vertébrale.

Ce matériel comprend également deux tiges d'étayage destinées à être disposées parallèlement l'une à l'autre de part et d'autre des vertèbres à traiter et des organes d'ancrage de ces tiges aux vertèbres, tels que des crochets ou des vis pédiculaires. Ces tiges et organes d'ancrage sont bien connus en eux-mêmes et ne sont donc pas particulièrement décrits.

Comme le montre la figure 1, la traverse 1 est en deux parties 5, 6. La partie 5 comprend une tête sphérique 7 au niveau de son extrémité destinée à être reliée à la partie 6 et un filetage 8 adjacent à cette tête 7. La partie 6 comprend une extrémité renflée 9 de forme sphérique, délimitant intérieurement une cavité sphérique 10 propre à recevoir la tête 7 avec possibilité de mouvements d'articulation de cette tête 7 dans cette cavité 10. La paroi 11 délimitant cette cavité 10 est sertie autour de la tête 7 de manière à assurer la rétention de cette tête dans la cavité 10.

La traverse 1 comprend également un écrou 12 engagé sur la partie 5, propre à coopérer avec le filetage 8. Cet écrou 12 présente une portée sphérique 13 de forme correspondant à celle de la paroi 11.

Chaque partie de traverse 5, 6 comprend en outre une ouverture oblongue 15 au travers de laquelle peut être engagée une vis 3, et une pluralité d'évidements 16 délimitant, individuellement ou par paires, des logements circulaires propres à recevoir la tête 3a de cette vis 3.

Chaque pièce 2 est monobloc et présente un évidement 20, une fente 21 a, 21 b en forme de "L" et deux alésages coaxiaux.

L'évidement 20 est aménagé dans la pièce 2, à partir d'un des bords de celle-ci, et traverse cette pièce 2. II est circulaire et a un diamètre légèrement supérieur à celui des tiges d'étayage, de telle sorte qu'il peut recevoir étroitement une tige d'étayage en lui. Il délimite ainsi deux parois de serrage latérales 25.

La fente en forme de "L" est aménagée au travers de la pièce 2 selon une même direction que l'évidement 20. Une première partie 21 a des deux parties 21 a, 21 b de cette fente débouche dans l'évidement 20, latéralement par rapport à celui-ci, et est orientée de manière sensiblement parallèle aux parois 25. La deuxième partie 21 b de fente est orientée sensiblement perpendiculairement aux parois 25 et s'étend sur une longueur relativement importante. II en résulte que chaque paroi 25 est reliée au reste de la pièce 2 par une zone 26 de largeur relativement réduite, présentant une souplesse élastique, et que cette fente définit deux zones 27, 28 de la pièce 2, à savoir une zone 27 délimitée par la face de la pièce 2 opposée à l'évidement 20 et par la partie de fente 21 b, et une zone 28 délimitée par la partie de fente 21 b et par l'évidement 20.

Parmi les deux alésages précités, l'un est aménagé au travers de la zone 27 et n'est pas taraudé tandis que l'autre est aménagé au travers de la zone 28 et est taraudé.

La vis 3 présente quant à elle une tête 3a lui permettant de prendre appui contre les parties 5, 6 au niveau des évidements 16 et un corps pouvant être engagé dans les ouvertures 15 et dans l'alésage non taraudé précité d'une pièce 2, puis pouvant être vissé au travers de l'alésage taraudé de cette pièce 2.

La tête 7 et la cavité 10 forment une rotule permettant un positionnement multidirectionnel d'une partie de traverse 5, 6 par rapport à l'autre, de sorte que la traverse 1 peut ainsi s'adapter à toutes les situations susceptibles de se présenter, notamment en ce qui concerne l'écartement et/ou l'orientation des tiges d'étayage. L'écrou 12 permet de créer une tension sur la tête 7 par rapport à l'extrémité 9 et des frottements qui permettent d'immobiliser une partie de traverse 5, 6 par rapport à l'autre.

Le serrage de chaque vis 3 provoque le rapprochement de la zone 28 et de la zone 27, par déformation de la pièce 2. Cette déformation permet le serrage des parois 25 autour d'une tige d'étayage engagée dans l'évidement 20.

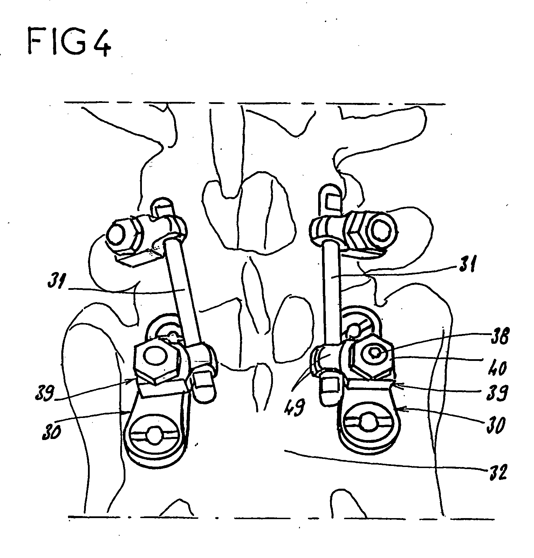

Comme le montre par ailleurs la figure 4, le matériel d'arthrodèse comprend en outre deux plaquettes 30 d'ancrage de tiges d'étayage 31 au niveau du sacrum 32.

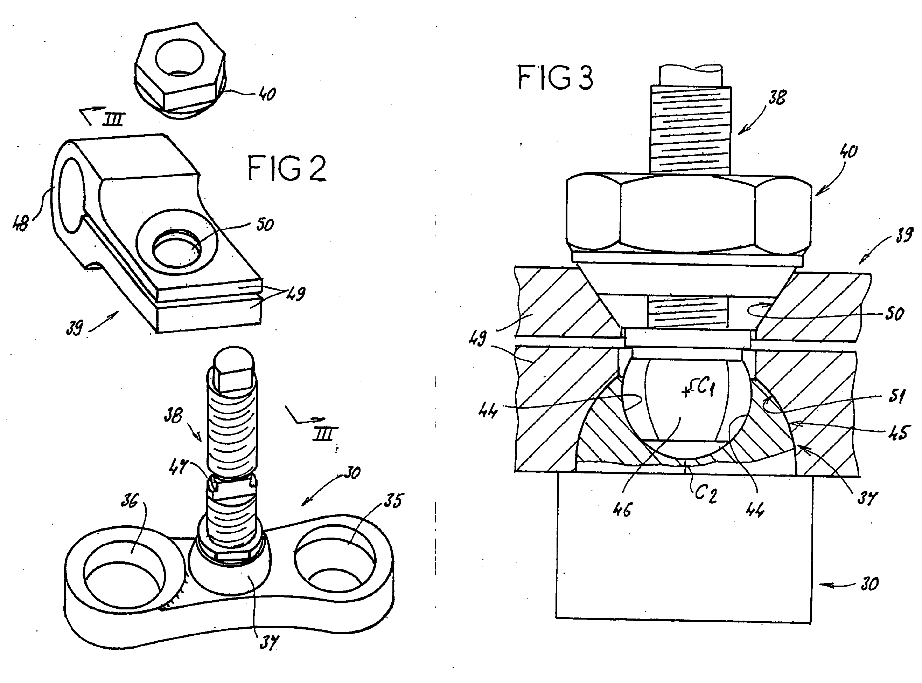

Il apparaît sur les figures 2 et 3 que chacune de ces plaquettes 30 présente deux trous latéraux 35, 36 pouvant recevoir des vis d'ancrage, et un bossage central 37 au niveau duquel est montée une tige filetée 38. Cette tige peut recevoir un étrier 39 et un écrou 40.

Chaque trou 35 a un axe sensiblement perpendiculaire à la face par laquelle la plaquette 30 est destinée à venir en appui contre le sacrum tandis que chaque trou 36 a un axe qui forme un angle d'environ 60 degrés par rapport à cette même face.

Le bossage 37 est solidaire de la plaquette 30. II délimite intérieurement, avec la plaquette 30, une cavité 44 de forme sphérique et délimite extérieurement une portion 45 de paroi sphérique.

Comme le montre la figure 3, les centres C1 générant la sphère creuse qui forme la cavité 44 et le centre C2 générant ladite portion de paroi sphérique 45 sont décalés l'un par rapport à l'autre selon une direction perpendiculaire à la plaquette 30.

La tige filetée 38 comprend une tête sphérique 46 destinée à être engagée et à être retenue dans la cavité 44. Cette tête 46 est propre à coopérer avec cette cavité 44 de manière à permettre l'articulation de la tige 38 par rapport à la plaquette 30.

La tige 38 présente également une portion amincie 47, permettant sa section après montage définitif du matériel.

L'étrier 39 présente une partie arrondie 48 propre à recevoir une tige d'étayage, deux ailes 49 superposées percées de trous 50 pour permettre l'engagement de cet étrier 39 sur la tige 38, et une face 51 en portion de sphère creuse. Cette face 51 est, ainsi que le montre la figure 3, destinée à venir porter contre la portion de paroi sphérique 45 lorsque l'étrier 39 est engagé sur la tige 38.

L'écrou 40 peut être vissé sur la tige 38 de manière à permettre le serrage de l'étrier 39 entre lui et ladite portion de paroi sphérique 45.

Grâce à la cavité 44 et à la tête sphérique 46, la tige 38 peut, au moment de la mise en place du matériel, être orientée en direction de l'étrier 39, lui-même préalablement engagé autour de la tige d'étayage. Cette orientation permet de faciliter l'engagement des trous 50 de l'étrier 39 sur cette tige 38.

L'écrou 40 peut alors être engagé sur la tige 38 et être vissé, ce qui a pour effet d'amener ladite face 51 de l'étrier 39 au contact de ladite portion de paroi sphérique 45 puis, une fois ce contact intervenu, et compte tenu du décalage des deux centres C1, C2 précités, d'amener progressivement, au fur et à mesure du serrage de l'écrou 40, la tige 38 dans une direction sensiblement perpendiculaire à la plaquette 30.

L'invention fournit ainsi un matériel d'arthrodèse vertébrale présentant les avantages importants précités par rapport aux matériels homologues de la technique antérieure.

II va de soi que l'invention n'est pas limitée à la forme de réalisation décrite ci-dessus à titre d'exemple mais qu'elle en embrasse au contraire toutes les variantes de réalisation couvertes par les revendications ci-annexées.

## Revendications

1. Matériel d'arthrodèse vertébrale, comprenant deux tiges d'étayage destinées à être disposées parallèlement l'une à l'autre de part et d'autre des vertèbres à traiter, des organes d'ancrage de ces tiges aux vertèbres, tels que des crochets ou des vis pédiculaires, au moins une traverse (1) en deux parties (5, 6) et des organes (2) d'assemblage de cette traverse (1) aux tiges d'étayage ;
matériel **caractérisé en ce que** :
- l'une des parties de traverse (5) comprend une tête sphérique (7) au niveau de son extrémité destinée à être reliée à l'autre partie de traverse (6) et un filetage (8) adjacent à cette tête (7) ;
- l'autre partie de traverse (6) comprend une extrémité renflée (9) de forme sphérique, délimitant intérieurement une cavité sphérique (10) propre à recevoir ladite tête (7) avec possibilité de mouvements d'articulation de cette tête (7) dans cette cavité (10), la paroi (11) délimitant cette cavité (10) étant sertie autour de ladite tête (7) de manière à assurer la rétention de cette tête (7) dans la cavité (10), et
- chaque traverse (1) comprend un écrou (12) propre à coopérer avec le filetage (8) précité, présentant en-outre une portée sphérique (13) de forme correspondant à celle de ladite paroi (11) délimitant la cavité (10).

2. Matériel selon la revendication 1, **caractérisé en ce qu'**il comprend une vis (3) pour relier une partie de traverse (5, 6) à l'organe (2) permettant l'assemblage de cette partie de traverse (5, 6) à la tige d'étayage correspondante, et **en ce que** cette partie de traverse (5, 6) comprend une ouverture oblongue (15) permettant une pluralité de positions de cette vis (3), et donc de l'organe d'assemblage (2), par rapport à la partie de traverse (5, 6).

3. Matériel selon la revendication 2, **caractérisé en ce que** chacune des deux parties de traverse (5, 6) comprend une ouverture oblongue (15) permettant une pluralité de positions de la vis (3) par rapport à la partie de traverse (5, 6).

4. Matériel selon la revendication 2 ou la revendication 3, **caractérisé en ce que** chaque partie de traverse (5, 6) comprenant ladite ouverture oblongue (15) présente une pluralité d'évidements (16) délimitant, individuellement ou par paires, des logements circulaires propres à recevoir la tête (3a) de ladite vis (3).

5. Matériel selon l'une des revendications 1 à 4, **caractérisé en ce que** chaque organe d'assemblage comprend une pièce monobloc (2) présentant :
- un évidement (20) aménagé dans cette pièce (2), à partir d'un des bords de celle-ci, qui traverse la pièce (2) et qui délimite deux parois de serrage (25) latérales, propres à recevoir étroitement une tige d'étayage entre elles ;
- une fente (21 a, 21 b) en forme de "L", aménagée au travers de cette pièce (2) selon une même direction que ledit évidement (20) ; une première (21 a) des deux parties de fente (21 a, 21 b) débouche dans l'évidement (20), latéralement par rapport à celui-ci, et est orientée de manière sensiblement parallèle auxdites parois de serrage (25) ; la deuxième partie de fente (21 b) est orientée sensiblement perpendiculairement auxdites parois de serrage (25) et s'étend sur une longueur relativement importante, de telle sorte que chaque paroi de serrage (25) est reliée au reste de la pièce (2) par une zone (26) de largeur relativement réduite, présentant une souplesse élastique, et
- deux alésages coaxiaux, dont un premier, non taraudé, est aménagé au travers d'une première zone (27) de la pièce (2), délimitée par la face de cette pièce (2) opposée à l'évidement (20) d'une part et par ladite deuxième partie de fente (21 b) d'autre part, et dont un deuxième, taraudé, est aménagé au travers d'une deuxième zone (28) de la pièce (2), délimitée par ladite deuxième partie de fente (21 b) d'une part et par l'évidement (20) d'autre part.

6. Matériel selon l'une des revendications 1 à 5, **caractérisé en ce qu'**il comprend deux plaquettes (30) d'ancrage de tiges d'étayage (31) au niveau du sacrum (32), comportant chacune des moyens d'assemblage à une tige d'étayage (31) correspondante ;
- chaque plaquette (30) présente deux trous latéraux (35, 36) pouvant recevoir des vis d'ancrage, l'un de ces trous (35) ayant un axe sensiblement perpendiculaire à la face par laquelle cette plaquette (30) est destinée à venir en appui contre le sacrum (32) et l'autre trou (36) ayant un axe qui forme un angle d'environ 60 degrés par rapport à cette même face ;
- les moyens d'assemblage que comporte chaque plaquette (30) comprennent :
(i) un bossage (37) solidaire de la plaquette (30), délimitant intérieurement, avec la plaquette (30), une cavité (44) de forme sphérique et délimitant extérieurement une portion de paroi sphérique (45) ; les centres (C1, C2) générant la sphère creuse qui forme la cavité (44) et ladite portion de paroi sphérique (45) sont décalés l'un par rapport à l'autre selon une direction perpendiculaire à la plaquette (30) ;
(ii) une tige filetée (38) comprenant une tête sphérique (46) destinée à être engagée et à être retenue dans ladite cavité (44) de la plaquette (30), cette tête (46) étant propre à coopérer avec la cavité (44) de manière à permettre l'articulation de la tige filetée (38) par rapport à la plaquette (30) ;
(iii) un étrier (39) présentant une partie arrondie (48) propre à recevoir une tige d'étayage, deux ailes (39) superposées percées de trous (50) pour permettre l'engagement de cet étrier (39) sur ladite tige filetée (38), et une face (51) en portion de sphère creuse, cette face (51) étant destinée à venir porter contre ladite portion de paroi sphérique (45) lorsque l'étrier (39) est engagé sur ladite tige filetée (38), et
(iv) un écrou (40) pouvant être vissé sur la tige filetée (38) de manière à permettre le serrage de l'étrier (39) entre lui et ladite portion de paroi sphérique (45).

7. Matériel selon la revendication 6, **caractérisé en ce que** la tige fileté (38) présente une portion amincie (47), permettant sa section après montage définitif du matériel.

## Claims

1. Vertebral arthrodesis equipment, comprising two shoring rods intended to be placed parallel to each other on either side of the vertebrae to be treated, anchoring members to attach these rods to the vertebrae, such as hooks or pedicular screw (3)s, at least one cross-piece (1) in two-part (5, 6), and attachment members (2) to attach this cross-piece (1) to the shoring rods,
**characterized in that:**
- one of the cross-piece parts (5) comprises a spherical head (7) (7) at its extremity intended to be connected to the other part (6) of the cross-piece (1), and a thread (8) adjacent to this head (7) (7) ;
- the other cross-piece part (6) has a spherical bulging end (9), defining internally a spherical cavity (10) (10) adapted to receive said head (7), with a possibility of movement of this head (7) within this cavity (10), the wall (11) delimiting the cavity (10) being crimped around said head (7) in such a way as to retain this head (7) within the cavity (10), and
- each cross-piece (1) comprises a nut (12) designed to cooperate with said thread (8), including further a spherical seat (13) matching that of said wall (11) delimiting the cavity (10).

2. Equipment according to claim 1, **characterized in that** it includes a screw (3) to connect one of the cross-piece parts (5, 6) to the member (2) that enables the attachment of this part (5, 6) of the cross-piece to the corresponding shoring rod, and **in that** this part (5, 6) of the cross-piece includes an oblong opening (15) allowing a plurality of positions for this screw (3), and thus for the attachment member (2), relative to that part (5, 6) of the cross-piece.

3. Equipment according to claim 2, **characterized in that** each of the cross-piece parts (5, 6) has an oblong opening (15) enabling a plurality of positions for this screw (3) relative to that part (5, 6) of the cross-piece.

4. Equipment according to claim 2 or claim 3, **characterized in that** each cross-piece part (5, 6) comprising said oblong opening (15) has several recesses (16) which, either individually or in pairs, form circular seatings designed to receive the head (3a) of the aforementioned screw (3).

5. Equipment according to claims 1 to 4, **characterized in that** each attachment member includes a one-piece member (2) that has:
- a recess (20) in the member (2), formed as from the edges thereof, which crosses the member (2) and forms two lateral clamping walls (25), for fittingly receiving a shoring rod between them;
- an L-shaped slot (21 a, 21 b) formed through said member (2) in the same direction as said recess (20) ; one part (21 a) of the two parts (21 a, 21 b) of the L-shaped slot opens laterally into the recess (20), and is essentially parallel to the clamping walls (25) ; the other part (21 b) of the slot is in an essentially perpendicular direction to the clamping walls (25) and is relatively long, so that each clamping wall (25) is connected to the rest of the member (2) by a relatively narrow section (26,) thereby offering elasticity, and
- two coaxial bores, a first of which does not have a thread and is formed through a first part (27) of the member (2), defined by the face of this member (2) opposite to the recess (20) and by said second part (21 b) of the L-shaped slot, and the second, with a thread, is formed through a second part (28) of the member (2), defined, on one hand, by said second part (21 b) of said L-shaped slot and by the recess (20) on the other hand.

6. Equipment according to claims 1 to 5 **characterized in that** it comprises two fixing plates (30) to attach the shoring rods (31) to the sacrum (32), each having attachment means to the corresponding shoring rod (31) ;
- each plate (30) has two lateral holes (35, 36) able to receive fixing screws, one of the holes (35) having an axis essentially perpendicular to the face by which the plate (30) is to be mounted onto the sacrum (32), and the other hole (36) having an axis at an angle of about 60 degrees relative to this same face ;
- the attachment means of each plate (30) comprises :
(i) a domed part (37) of the plate (30), internally forming, with the plate (30), a spherical cavity (44) and externally forming a spherical surface (45) ; the centres (C1, C2) generating the hollow sphere which forms said cavity (44) and said spherical surface (45) being offset one relative to the other in a direction perpendicular to the plate (30) ;
(ii) a threaded rod (38) with a spherical head (46) that fits into said cavity (44) and can be held therein, this head (46) being designed to cooperate with the cavity (44) in such a way to allow movement of the rod (38) relative to the plate (30) ;
(iii) a stirrup (39) with a rounded part (48) able to receive a shoring rod, and two flanges (39) with superimposed holes (50) to enable this stirrup (39) to be attached said threaded rod (38) ; and a surface (51) having a form of hollow sphere portion, this surface (51) being designed to bear against said spherical surface (45) when the stirrup (39) is mounted on the threaded rod (38), and
(iv)a nut (40) that may be tightened on the threaded rod (38) so as to enable the tightening of the stirrup (39) between the nut (40) and the spherical surface (45).

7. Equipment according to claim 6, **characterized in that** the threaded rod (38) has a thinner section (47) enabling the rod (38) to be cut once the equipment has been fitted.

## Patentansprüche

1. Ausstattung zur Wirbelarthrodese, umfassend zwei Stützstäbe, die dazu gedacht sind, parallel zueinander auf beiden Seiten der zu behandelnden Wirbel angeordnet zu werden, Organe zum Verankern dieser Stäbe an den Wirbeln, wie etwa Haken oder Pedikelschrauben, mindestens eine Querstrebe (1) aus zwei Teilen (5, 6) und Organe (2) zum Zusammenfügen dieser Querstrebe (1) mit den Stützstäben;
wobei die Ausstattung **dadurch gekennzeichnet ist, dass**:
- einer der Querstrebenteile (5) einen Kugelkopf (7) an seinem Ende, das dazu gedacht ist, mit dem anderen Querstrebenteil (6) verbunden zu werden, und ein Gewinde (8), das an diesen Kopf (7) angrenzt, umfasst;
- der andere Querstrebenteil (6) ein kugelförmiges bauchiges Ende umfasst, das innen einen Kugelhohlraum (10) begrenzt, der dazu geeignet ist, den Kopf (7) aufzunehmen, und zwar mit der Möglichkeit von Gelenkbewegungen dieses Kopfes (7) in diesem Hohlraum (10), wobei die Wand (11), die diesen Hohlraum (10) begrenzt, um den Kopf (7) herum gebördelt ist, um die Halterung dieses Kopfes (7) in dem Hohlraum (10) sicherzustellen, und
- jede Querstrebe (1) eine Mutter (12) umfasst, die dazu geeignet ist, um mit dem genannten Gewinde (8) zusammenzuwirken, und die ferner einen Kugelansatz (13) aufweist, dessen Form derjenigen der Wand (11) entspricht, die den Hohlraum (10) begrenzt.

2. Ausstattung nach Anspruch 1, **dadurch gekennzeichnet, dass** sie eine Schraube (3) umfasst, um einen Querstrebenteil (5, 6) mit dem Organ (2) zu verbinden, was das Zusammenfügen dieses Querstrebenteils (5, 6) mit dem entsprechenden Stützstab ermöglicht, und dass dieser Querstrebenteil (5, 6) eine längliche Öffnung (15) umfasst, die eine Vielzahl von Positionen dieser Schraube (3), und somit des Organs (2) zum Zusammenfügen, im Verhältnis zu dem Querstrebenteil (5, 6) ermöglicht.

3. Ausstattung nach Anspruch 2, **dadurch gekennzeichnet, dass** jeder der beiden Querstrebenteile (5, 6) eine längliche Öffnung (15) umfasst, die eine Vielzahl von Positionen der Schraube (3) im Verhältnis zum Querstrebenteil (5, 6) ermöglicht.

4. Ausstattung nach Anspruch 2 oder 3, **dadurch gekennzeichnet, dass** jeder Querstrebenteil (5, 6), der die längliche Öffnung (15) umfasst, eine Vielzahl von Aussparungen (16) aufweist, die einzeln oder paarweise kreisförmige Aufnahmen begrenzen, die dazu geeignet sind, den Kopf (3a) der Schraube (3) aufzunehmen.

5. Ausstattung nach einem der Ansprüche 1 bis 4, **dadurch gekennzeichnet, dass** jedes Organ zum Zusammenfügen ein einteiliges Stück (2) umfasst, das folgendes aufweist:
- eine Aussparung (20), die in diesem Stück (2) von einem seiner Ränder ausgehend eingerichtet ist, die durch das Stück (2) hindurch geht und die zwei seitliche Klemmwände (25) begrenzt, die dazu geeignet sind, einen Stützstab dazwischen fest aufzunehmen;
- einen L-förmigen Schlitz (21 a, 21 b), der durch dieses Stück (2) hindurch in derselben Richtung wie die Aussparung (20) eingerichtet ist; ein erster (21 a) der beiden Schlitzteile (21 a, 21 b) mündet in die Aussparung (20), im Verhältnis dazu seitlich, und ist im Wesentlichen parallel zu den Klemmwänden (25) ausgerichtet; der zweite Schlitzteil (21 b) ist im Wesentlichen senkrecht zu den Klemmwänden (25) ausgerichtet und erstreckt sich über eine relativ große Länge, so dass jede Klemmwand (25) mit dem Rest des Stücks (2) über einen relativ schmalen Bereich (26), der eine gewisse elastische Nachgiebigkeit aufweist, verbunden ist, und
- zwei koaxiale Bohrungen, von denen eine erste Bohrung ohne Gewinde durch einen ersten Bereich (27) des Stücks (2) hindurch eingerichtet ist, der einerseits durch die Seite dieses Stücks (2), die der Aussparung (20) gegenüberliegt, und andererseits durch den zweiten Schlitzteil (21 b) begrenzt ist, und von denen eine zweite Bohrung mit Gewinde durch einen zweiten Bereich (28) des Stücks (2) hindurch eingerichtet ist, der einerseits durch den zweiten Schlitzteil (21 b) und andererseits durch die Aussparung (20) begrenzt ist.

6. Ausstattung nach einem der Ansprüche 1 bis 5, **dadurch gekennzeichnet, dass** sie zwei Plättchen (30) zum Verankern von Stützstäben (31) auf Kreuzbeinhöhe (32) umfasst, die jeweils Mittel zum Zusammenfügen mit einem entsprechenden Stützstab (31) umfassen;
- jedes Plättchen (30) weist zwei seitliche Löcher (35, 36) auf, die Verankerungsschrauben aufnehmen können, wobei eines dieser Löcher (35) eine Achse aufweist, die zu der Seite, durch die dieses Plättchen (30) am Kreuzbein (32) zur Anlage kommen soll, im Wesentlichen senkrecht ist, und das andere Loch (36) eine Achse aufweist, die einen Winkel von ungefähr 60 Grad zu derselben Seite bildet;
- die Mittel zum Zusammenfügen, die jedes Plättchen (30) umfasst, folgendes umfassen:
(i) eine mit dem Plättchen (30) einstückige Erhebung (37), die innen mit dem Plättchen (30) einen kugelförmigen Hohlraum (44) begrenzt und außen einen Kugelwandabschnitt (45) begrenzt; wobei die Mittelpunkte (C1, C2), welche die hohle Kugel erzeugen, die den Hohlraum (44) bildet, und der Kugelwandabschnitt (45) zueinander in einer Richtung versetzt sind, die zu dem Plättchen (30) senkrecht steht;
(ii) einen Gewindestift (38), der einen Kugelkopf (46) umfasst, der dazu gedacht ist, mit dem Hohlraum (44) des Plättchens (30) in Eingriff gebracht und darin festgehalten zu werden, wobei dieser Kopf (46) dazu geeignet ist, mit dem Hohlraum (44) zusammenzuwirken, um die Drehbarkeit des Gewindestifts (38) im Verhältnis zu dem Plättchen (30) zu ermöglichen;
(iii) einen Bügel (39), der einen gerundeten Teil (48) aufweist, der dazu geeignet ist, einen Stützstab aufzunehmen, zwei übereinander liegende Flügel (39), die mit Löchern (50) versehen sind, um den Eingriff dieses Bügels (39) auf dem Gewindestift (38) zu ermöglichen, und eine Seite (51) in dem Hohlkugelabschnitt, wobei diese Seite (51) dazu gedacht ist, auf dem Kugelwandabschnitt (45) zu ruhen, wenn der Bügel (39) auf dem Gewindestift (38) in Eingriff steht, und
(iv) eine Mutter (40), die auf den Gewindestift (38) aufgeschraubt werden kann , um das Einklemmen des Bügels (39) zwischen dieser und dem Kugelwandabschnitt (45) zu ermöglichen.

7. Ausstattung nach Anspruch 6, **dadurch gekennzeichnet, dass** der Gewindestift (38) einen verjüngten Abschnitt (47) aufweist, der sein Durchschneiden nach dem endgültigen Einbau der Ausstattung ermöglicht.
